# EUROPEAN PATENT SPECIFICATION

(11) **EP 0 858 294 B1**
(45) Date of publication and mention of the grant of the patent: **07.11.2001**
(21) Application number: 96928574.1
(22) Date of filing: 30.08.1996
(51) Int. Cl.: A61B 17/34, A61B 10/00

(54) **MAGNETIC RESONANCE-COMPATIBLE NEEDLE**
EINE MIT MAGNETISCHER RESONANZ KOMPATIBLE NADEL
AIGUILLE COMPATIBLE AVEC L'IMAGERIE PAR RESONANCE MAGNETIQUE

(30) Priority: 31.08.1995 GB 9517781
(43) Date of publication of application: 19.08.1998
(73) Proprietor: UNIVERSITY COLLEGE LONDON, London WC1E 6BT (GB)
(72) Inventor: BROOKES, Jocelyn, Asher, Simon, London N2 8BH (GB)
(74) Representative: Jackson, Richard Eric
(86) International application number: PCT/GB96/02086
(87) International publication number: WO 97/07746

(56) References cited:
- EP-A- 0 174 011
- EP-A- 0 566 359
- WO-A-96/14023
- US-A- 5 290 266
- D5 Tables of Physical and Chemical constants by Kay and Laby publisher Longmans 1966 pages 2 , 94 , 102 , 107

## Description

Magnetic Resonance Imaging (MRI) scanning is a method of imaging the human body or other fleshy objects e.g. animals, either alive or dead. It depends on the object being placed in a large and uniform magnetic field (commonly ranging from 0.2-7.0 Tesla field strength according to the particular machine used).

The image is created by disturbance of molecules of the body by radiofrequency (RF) pulses causing resonance of these molecules. After the RF pulse, the molecules realign with the magnetic field and in so doing, emit a weak RF signal of their own. This signal is detected and analysed by a computer and a signal map created of the interrogated body part such that a greyscale image is displayed.

The appearance is usually that of 'slices' through the body revealing the inner organs. It is useful in medical practise, to place needles in these inner organs in the presence of disease, both to extract tissue and fluid samples for analysis (biopsy) or to pass fluids or instruments down the needles to alter the tissue of interest in a variety of prespecified ways.

The precise position of the needle tip is clearly vital for accurate diagnosis and/or therapy in this context.

To use a needle in an MRI scanner, the needle must fulfil all the requirements for a medicinal needle in terms of strength and body compatibility as well as being compatible with the MRI imaging apparatus in that it must be both clearly visible when scanned as well as causing no distortion artefact to the final image. Any magnetic object will distort the magnetic field and thus the image, making accurate placement difficult.

Such needles as are used currently, are made of metal alloys with high nonferrous metal content and a low relative proportion of Iron but these still cause significant artefact especially at the needle tip-the most important part.

EP-A-0174011 discloses a medical puncture needle moulded with a mixture of thermoplastic resin and fibrous material dispersed therein. EP-A-0566359 discloses an illuminated surgical trocar which includes a fiber reinforced plastic cannular, and a method for wrapping the fibres to make the cannular, and US 5,290,266 disclose a flexible coating for magnetic resonance imaging compatible invasive devices. The coating retains the hard material forming the device, in the event of a fracture.

In view of the foregoing, the present invention provides a magnetic resonance imaging (MRI) needle made of a material which is compatible for use in an MRI scanner, characterised in that the material is a carbon fibre-composite and causes no significant artefact or image distortion when visualised with an MRI scanner.

Preferably the fibres of the carbon fibre composite are arranged either axially, spirally, in circular fashion or in a combination of these orientations.

Preferably the needle is sterilizable by heat, chemicals and/or irradiation techniques allowing re-use of the needle.

A specific embodiment of the invention is now described by way of example with reference to the accompanying drawings. in which:-

Figure 1 shows the side view of the needle and a selection of accompanying components.

Figure 2 shows the needle end-on

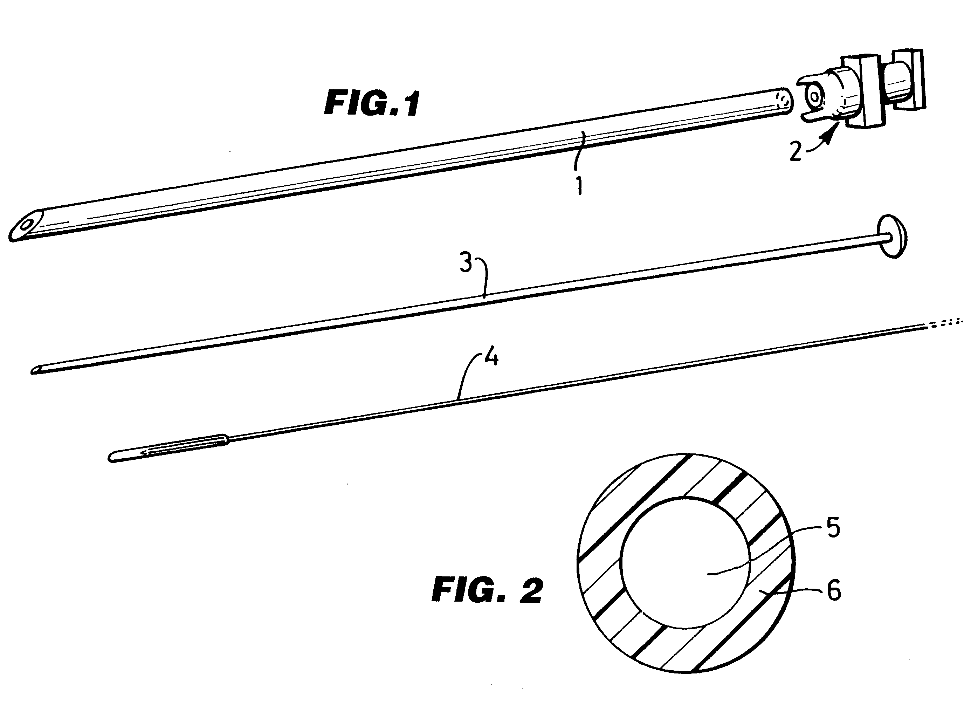

Referring to the drawings Figures 1 and 2, the needle comprises an outer cylindrical tube 1 of carbon fibre material with length and wall dimensions 5 and 6 variable according to the task required i.e.large or small needles.

The needle has a connector 2 attached to its proximal end to allow the connection of other apparatus.

The needle bore 5 allows passage of fluids in either direction, the insertion of an obturator, mandril or trocar 3 , as well as the passage of implements such as optical light fibres 4 , catheters and more complex instruments such as ultrasound probes

The needle and trocar assembly can be adapted to perform core biopsies of tissues as is currently undertaken with metallic needles.

The carbon fibre tubing is manufactured either by 'pultrusion' of the fibres over a mandril with the fibres arranged both axially and spirally, or wrapping preformed carbon fibre sheeting around a mandril with subsequent polishing of the surfaces.

It will of course be understood that the present invention has been described above purely by way of example, and that modifications of detail can be made within the scope of the claims.

## Claims

1. A magnetic resonance imaging (MRI) needle (1) made of a material which is compatible for use in an MRI scanner, **characterised in that** the material is a carbon fibre-composite and causes no significant artefact or image distortion when visualized with an MRI scanner.

2. A needle according to claim 1, wherein the carbon fibre is either of wrapped or of axially and/or spirally pultruded manufacturing configuration.

3. A needle according to any preceding claim, wherein the needle is sterilizable by heat or chemical or irradiation means such that it may be re-used.

## Patentansprüche

1. Magnetresonanz-Abbildungsnadel (MRI) (1), die aus einem Material gefertigt ist, welches für eine Verwendung in einem MRI-Scanner kompatibel ist, **dadurch gekennzeichnet, daß** das Material ein Kohlenstoffaserverbundmaterial ist und kein signifikantes Artefakt oder keine Bildstörung bzw. -verzerrung bewirkt, wenn es mit einem MRI-Scanner visualisiert wird.

2. Nadel nach Anspruch 1, worin die Kohlenstoffaser entweder aus einer umhüllten oder aus einer axial und/oder spiralig zieh- bzw. stranggepreßten Herstellungskonfiguration besteht.

3. Nadel nach einem der vorhergehenden Ansprüche, worin die Nadel durch Wärme bzw. Hitze oder chemische oder bestrahlende Mittel bzw. Strahlungsmittel sterilisierbar ist, so daß sie wiederverwendet werden kann.

## Revendications

1. Aiguille d'imagerie par résonance magnétique (MRI) (1) réalisée en un matériau qui est compatible pour l'utilisation avec un scanner MRI, **caractérisée en ce que** le matériau est un composite à base de fibres de carbone et ne provoque pas de parasite ou de distorsion d'image important lorsqu'elle est visualisée avec un scanner MRI.

2. Aiguille selon la revendication 1, dans laquelle la fibre de carbone est d'une configuration de fabrication soit par enroulement soit par pultrusion axiale et/ou en spirale.

3. Aiguille selon l'une quelconque des revendications précédentes, dans laquelle l'aiguille peut-être stérilisée par un moyen thermique, chimique ou d'irradiation de telle sorte qu'elle peut être réutilisée.
